# EUROPEAN PATENT SPECIFICATION

(11) **EP 2 950 743 B1**
(45) Date of publication and mention of the grant of the patent: **06.09.2017**
(21) Application number: 14707224.3
(22) Date of filing: 27.01.2014
(51) Int. Cl.: A61F 5/01, A61C 7/08

(54) **INTRAORAL DEVICE FOR IMPROVING PHYSICAL BALANCE**
INTRAORALE VORRICHTUNG ZUR VERBESSERUNG DES PHYSISCHEN GLEICHGEWICHTS
DISPOSITIF INTRA-BUCCAL DESTINÉ À AMÉLIORER L'ÉQUILIBRE PHYSIQUE

(30) Priority: 31.01.2013 IT RM20130061
(43) Date of publication of application: 09.12.2015
(73) Proprietor: Zampino, Claudio, 00153 Roma (IT)
(72) Inventor: Zampino, Claudio, 00153 Roma (IT)
(74) Representative: Fiammenghi, Eva
(86) International application number: PCT/IB2014/058580
(87) International publication number: WO 2014/118689

(56) References cited:
- US-A- 5 313 960
- US-A1- 2010 263 676
- US-A1- 2011 129 786

## Description

### Field of the art

The present invention refers to a new and innovative type of intraoral bite to be applied to the upper portion of the oral cavity. More in detail, the intraoral bite, object of the invention, comprises components adapted to facilitate an improved postural balance, as well as prevent the clenching of the premolars and the molars, generally involved when using conventional bites. These components induce a more suitable stimulation of the postural receptors of the palate, and prevent muscular contractions which can generate contractures and consequent dysfunctional responses.

### State of the art

A correct stabilization of the mandibular position allows an improved postural balance, when still and while walking, and particularly in athletic activities, with consequent reduction of the muscle-joint injuries and with improved sport performances.

The conventional bites such as those disclosed in US5313960 or US2010/0263676 are represented by a removable intraoral plate which is generally applied to the lower teeth. Substantially, these devices can be divided into: release plates, which have the object of preventing any occlusal interference of the mandible capable of conditioning the movement; repositioning plates, which confer the mandible, after having "deprogrammed" it, with a more defined and correct position; and stabilization plates, which serve to consolidate the new and correct position acquired with the previously used bites.

Independent of their specific purpose, the bites mainly serve to modify the mating between maxillary and mandibular dental arch, in order to find a lost position or to seek a possibility for neuromuscular and joint healing or reconditioning. For such purpose, the main psychophysical alterations, which normally accompany craniomandibular dysfunction are the following: cephalea; hemicrania; dizziness; earaches; tinnitus; sight disturbances; pharyngeal pain; pain; paresthesias and facial spasms; mandibular contractures; snap movements and temporomandibular pain; tooth hypersensitivity; parafunctions (onychophagia, nighttime and/or daytime clenching of the teeth); psychophysical fatigue (chronic tiredness); light fevers; cervicalgia; arthromuscular problems due to the rachis and the limbs responsible for disturbances of postural regulation. In particular for the latter type of disturbances, which are substantially translated into an incorrect posture, recent studies have shown that in the oral cavity, and particularly in the palate a great many receptors are present that are involved in the postural information mechanism. More in detail, the palatal stimulation is capable of affecting the operation of the primary postural receptors from the eye to the foot, the vestibular apparatus and the mandible. The general myorelaxant effect and the rebalancing effect on the musculature of the entire body, determined by the palatal stimulation, has been observed. The lingual action on the palate interacts on the trigeminal nerve: the stimulation of the palate by the tongue determines various physiological responses that involve the Central Nervous System and the *locus ceruleus.* In particular, the tongue, by compressing the palate receptors, is capable of indirectly inducing a refunctionalization of the Central Nervous System. It is also known that the change of the mandibular position obtained orthodontically or through the use of bites is sometimes capable of improving the subject's postural alignment. This is undoubtedly due to more suitable trigeminal information that can be found when the mandible assumes a position of neuromuscular balance. Of fundamental importance is, in particular, the coherence of the information that departs from the trigeminal nerve. The entire stomatognathic apparatus indeed has a trigeminal innervation. Trigeminal are the periodontal receptors and the information starting from the neuromuscular spindles of the masseters. These fibers are distinguished by an extremely rapid conduction, necessary for the CNS need to be informed with maximum urgency on the state of the masticatory action and occlusion. The information that derives therefrom is rapid but extremely transient, given that it is immediately substituted by subsequent information. The information starting from the palate spot is instead slower; it passes through several different paths (rather than directly reaching the *Locus ceruleus,* through the Reticular Substance and the trigeminal nuclei), but it has longer duration, such that the benefits of a stimulation of brief duration sometimes persist for a longer time.

The complete activation of the trigeminal nerve occurs only in one occasion: at the time of deglutition. Indeed, during deglutition there is the stimulation of the nasopalatine spot, the maxillary and mandibular periodontal receptors as well as the spindle receptors.

The posture is considerably sensitive to the stimulation of the palatine receptor. In particular, the results of many studies conducted in this field have shown that a suitable palatine trigeminal stimulation determines improvements of the plantar support in all components thereof.

The tongue which does not contact the palate, and which induces an incorrect deglutition, is therefore capable of interfering with all these receptor systems. In particular, an absent or incorrect palatal stimulation can give rise to altered mandibular postures; it can affect the eye in its sight capacity, due to the cervical alterations (the ciliary nerve has origin at the cervical level and determines the focusing of the crystalline lens). The deglutition dysfunction, caused by an incorrect palatal stimulation by the tongue, is also capable of altering the eye receptor through the variations of air pressure determined by the incorrect deglutition, with probable variation of the quality of the endolymph and the rolling of the otoliths.

The stimulation of the palate also acts on the foot with muscular mechanism. Upon baropodometric examination, it is frequent to see a pes cavus assume a more physiological support mode by simply flattening the palatine spot, due to the rebalancing of the tensions. The device, described hereinbelow, applied to the upper portion of the oral cavity can supply:
- information on the morphofunctional alignment of the craniomandibular complex;
- a general analysis on the static and dynamic postural appearance, with particular reference to the baropodometric exam;
- the possibility to analyze the postural parameters by varying the thickness of the front block, "plate", in the areas of the canines, upper and lower incisors;
- the analysis of the dynamic and static vestibular portion;
- the possibility to observe incorrect positions of the musculoskeletal-craniomandibular complex;
- the possibility of an improved physiological performance of the person.

The device provides for the possibility to graduate the height of the plate which can emphasize the following: functional alterations of various type relative to pain syndromes of different severity, and their localization, in particular, in the trigeminal, cervical and spinal - sensory district. Often such pathologies can be considered as referable to facial asymmetries. The possibility to graduate the height of the plate can also serve for a morphofunctional evaluation that can be useful for acquiring altered physiological data. In addition, it can be of great benefit to all those who practice sports activities (and are thus subjected to demanding loads), to children obliged to stay for hours in unhealthy positions on school desks or at the computer, and to the elderly who have various pseudopathological-dysfunctional problems. Recent studies of the neurobiologist Eric R. Kandel - Nobel Prize winner in medicine in 2000 due to his research on the biochemical mechanisms that lead to the formation of nerve cell memory - have underlined the importance of a good facial symmetry in living beings, and especially in humans.

Kandel refers the studies of David Perrett, Director of the Perception Laboratory of the University of St. Andrews in Scotland, which prove how empirical research has demonstrated that, throughout various cultures, woman and men prefer symmetric faces. This principal is deemed at the base of the selection for the coupling not only of human beings and higher apes, but also of birds and even insects. The reason for which this "bilateral prejudice" has been so well preserved throughout the animal kingdom is, according to Perrett - supported in this intuition by Nobel Prize winner Kandel - ascribable to the fact that good symmetry would indicate good genes. During growth, health problems and environmental stress factors can lead to asymmetrical growth models of the face. The degree of symmetry in the face of a person can thus indicate how well the genome of a person is capable of resisting disease and maintaining normal development in the face of challenges. In addition, the stability of the development is mainly hereditary, such that the symmetry, at least for the face, is beautiful not only for strictly formal reasons, but also for that which it communicates regarding the health of a potential companion and on his/her potential children.

These scientific reflections support the principle underlying the oral device, which in its assemblable part, indicated herein as "plate", exerts a "remodeling" action on the craniomandibular apparatus, making it more symmetrical overall.

In light of all the aforesaid observations, the present invention proposes to provide a new type of intraoral devices, and in particular of bites adapted to ensure a constant and more physiological palatal stimulation and to induce a more symmetric craniomandibular realignment, with the result of finding clear improvements of the balance, posture and morphofunctional alignment of the craniomandibular complex of the subject using the device. These conditions are obtained due to a particular portion of the present device, which is extended, when applied inside the oral cavity of the patient, in a manner so as to constantly contact the front portion of the palate, simulating a more suitable lingual stimulation, where said stimulation is inefficient, and due to an assemblable component adapted to prevent the complete occlusion of the buccal bite, facilitating a more symmetric realignment of the mandibular apparatus. Said assemblable plate has variable thickness and is extended, when applied to the buccal system of the subject, from canine to canine. These characteristics ensure that neither molars nor premolars are involved in the rehabilitative process, contrary to what happens when using conventional bites. This prevents the occurrence of muscular contractions, which can then degenerate into contractures with consequent dysfunctional responses.

### Description of the invention

The present invention refers to a new and innovative intraoral device, belonging to the category of neurological bites, adapted to induce improvements in the postural balance of the subject using said device and morphofunctional alignment of the craniomandibular complex of said subject. This important, clear result is obtained due to the presence of a particular component, integrated with the device itself, which has the function of carrying out a constant and more physiological palatal stimulation, simulating lingual activity on the front portion of the palate. More in detail, the device that is the object of the present industrial invention patent application is first of all distinguished, with respect to the conventional bites, due to the fact that it is applicable to the upper portion of the oral cavity rather than to the lower portion. Still more in detail, the described intraoral bite comprises a sheath made of resin or similar material, covering at least the central portion of the upper dental arch and preferably the entire upper dental arch, where it is firmly integrated with said sheath, at the back of the upper incisors, a resin extension which is extended along the front portion of the palate. This extension, which represents the palatal stimulation component when said intraoral bite is worn by the patient, exerts a constant and mild pressure on the front portion of the palate, stimulating its receptors in a more suitable manner, if a disturbing dysfunction has been encountered at the postural level for which the use of the present device is recommended. This palatal stimulation component is also shaped and sized in a manner such to allow the mandible, when the present device is worn by the patient, to freely move and to make the front teeth of the lower dental arch occlude on a surface, represented by the component in question, which interferes in the clenching action of the teeth, preventing the wear of the tooth surfaces as well as the increase of noradrenalin production. In particular, the shape, size, location and extension of this palatal stimulation component allow maintaining the premolars and molars at rest; these will not be involved in buccal clenching, consequently preventing muscular contractions that, if overly prolonged, can generate contractures and relative dysfunctional responses.

The present intraoral device, whose comprehension will be clearer in the course of the present description, also due to the observation of the enclosed figures, further comprises at least one assemblable component adapted to induce improvements of peripheral physiological functions, altered by asymmetric dysfunctions encountered upstream at the facial level. More in detail the described intraoral device comprises at least one assemblable component represented by a resin plate, with variable thickness, to be assembled, if required, to the palatal stimulation component. The resin plate in question is characterized in that it is extended from canine to canine, when assembled to the present device, in turn applied to the buccal system of the patient. Analogous to the described palatal stimulation component, the assemblable plate carries out its function without involving the molars and premolars, thus preventing prolonged contractures and dysfunctional responses.

The plate in question, when assembled, thus facilitates the establishment of a mandibular realignment mechanism, if facial asymmetries are encountered that cause peripheral dysfunctions through the trigeminal nerve. This is without involving the molars and premolars, contrary to what occurs when using the conventional bites. Depending on the extent of the asymmetries encountered in the patient, the plate in question has a variable thickness. The possibility of substituting the plate by varying the thickness thereof confers high versatility to the present intraoral device, which makes it particularly effective for the establishing of a true rehabilitative physiologic path. For example, the plate can from time to time be substituted with plates of lower thickness until clear improvements of the symmetric mandibular alignment are seen in the subject. In adolescents and children, during psycho-neurological growth and development, the action of the bite can function as an element capable of restoring the morphofunctional balance in the most harmonic manner. Indeed, the human organism functions as a cybernetic system capable of being adapted, self-regulated and self-programmed. This means that based on the information received at each instant, from the external and internal environment, the system tends to constantly reach conditions of equilibrium, commonly termed homeostasis. Even if this represents a cybernetic system, it still encounters - like all systems of this type - regulation and programming errors. The higher the number of errors, the lower the number of signals and more in detail stimuli coming from the external environment. In other words, the more numerous and varied the external information that the organism receives, the more it is able to execute a fine and correct regulation of its operation. This means that by continuously changing the plate, of course depending on the case, with another plate with thickness different from that of the preceding plate, the organism will have the capacity to store and select the mandibular alignment corresponding to more physiological peripheral efferent responses.

The possibility of varying the plate also makes the use of the present device decidedly advantageous, since said use prevents changing the entire device each time the change is required, and instead obliges only the substitution of the plate with one of opportune thickness. It is important to note that the operation of the present intraoral device has greater effectiveness by accompanying the use of the device with suitable tests, such as stabilometric and/or baropodometric and/or vestibular tests that allow evaluating the extent of the problem and selecting the plate and/or plates of specific thickness.

### Brief description of the drawings

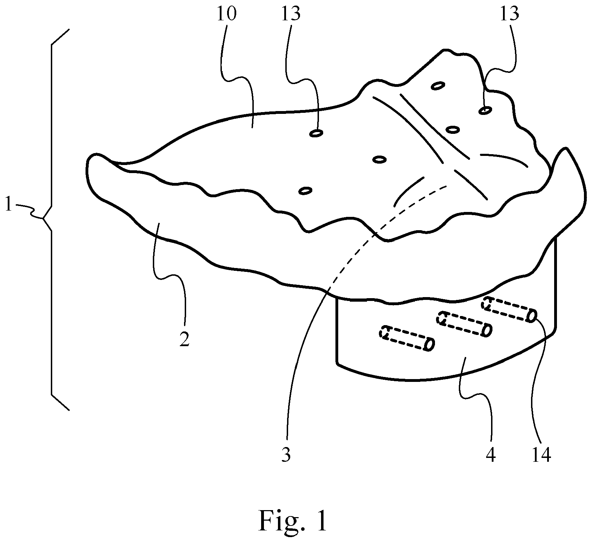

FIGURE 1 shows a perspective view of the intraoral device 1, according to the present invention, for improving postural balance of the subject using said device 1. Said intraoral device 1 is to be applied to the upper portion of the dental arch of the subject, and comprises: the sheath 2 adapted to cover at least one portion of the upper dental arch of the subject and preferably the entire upper dental arch of the subject, projecting therefrom in a manner so as to cover the palate of the subject, as in the case of the figure in question, in which the extended portion 10 can be observed which is adapted to contact the palate when the device 1 is applied to the buccal system of the subject. The intraoral device 1 further comprises the palate stimulation component 3 adapted to induce a more physiological stimulation of the palate receptors. Said component 3 constitutes a single body with the sheath 2, given that it is firmly fixed to the latter, and is extended along the front portion of the palate when said device 1 is applied to the subject. The component 3 carries out its function as palatal stimulator, exerting a mild pressure on the front portion of the palate, being extended directly on the back of the upper incisors when the device 1 is worn by the subject. In figure 1, it is also observed that the intraoral device 1, object of the present invention, comprises the plate 4 adapted to induce a realignment of the mandibular alignment of the subject, in the case in which asymmetries can be found involving the temporomandibular joint. The application of the plate 4 is optional and its thickness varies according to the extent of the asymmetry encountered. The figure in question also shows that the extended portion 10 has at least one hole 13 adapted to facilitate the respiration of the palate when the device 1 is worn by the subject. Also the plate 4 optionally has at least one channel 14 and preferably three channels 14 adapted to facilitate the respiration of the subject, in particular during sports fatigue.

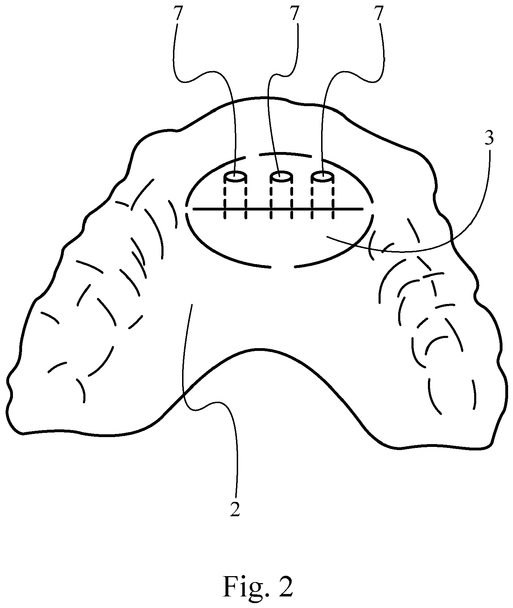

FIGURE 2 shows a view of the lower face of the sheath 2 of the described intraoral device 1, i.e. a view of the face directed towards the muscular floor of the buccal system of the subject. It is observed in the figure that the component 3, extended on the back of the upper incisors of the subject when the device 1 is worn, comprises at least one hole 7 and preferably three holes 7 adapted for the optional assembly of the plate 4 (not shown in the figure in question) to said component 3.

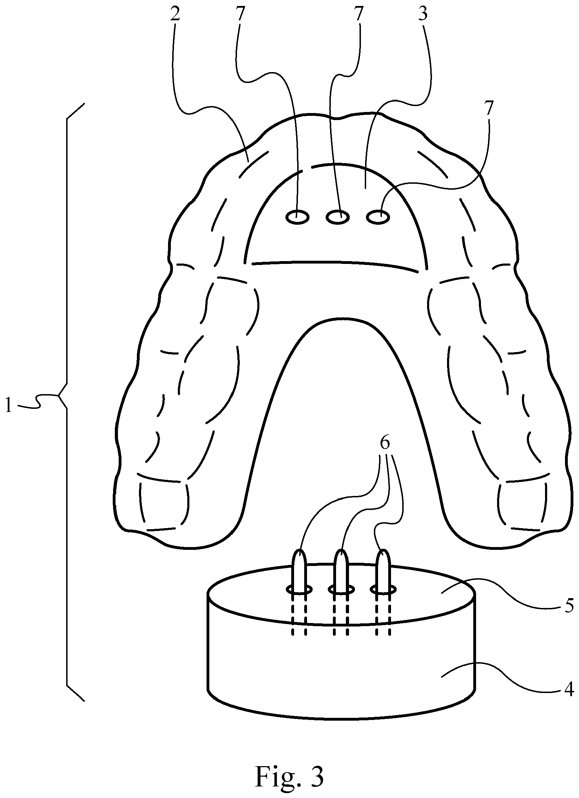

FIGURE 3 shows an exploded view of the intraoral device 1 according to the present invention. More in detail, figure 3 shows a plan view of the face, of the sheath 2, directed towards the muscular floor of the buccal system of the subject and a view of the component 4. Analogous to figure 2, figure 3 shows the presence of the holes 7 present on the component 3 adapted for the optional assembly of the component 4. The latter, as will be observed in the figure in question, comprises at least one pin 6 and preferably three pins 6 to be inserted in corresponding holes 7. Said pins 6 are in more detail inserted and fixed in the plate 4 and project from the face 5, of said plate 4, to be directed towards the palate of the subject using the device 1.

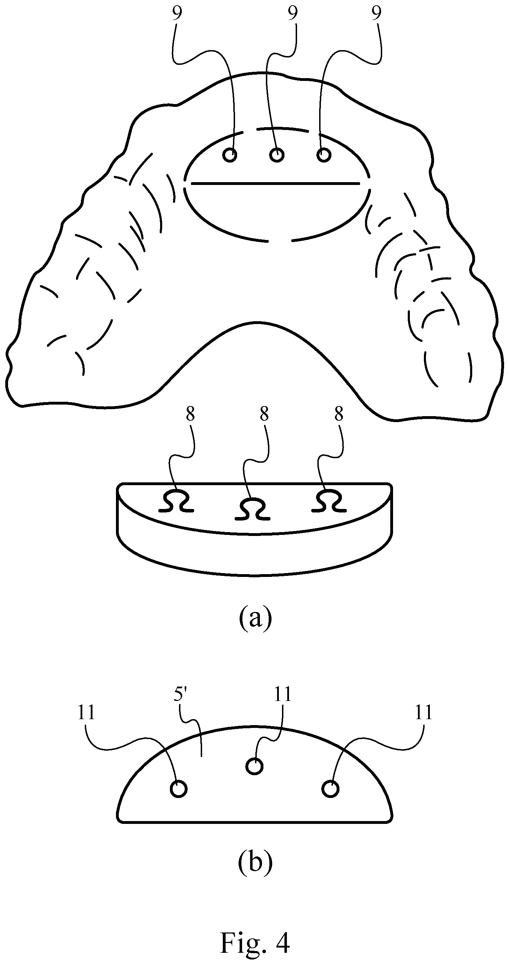

FIGURE 4 shows a view of a particular embodiment of the device 1 according to the present invention. More in detail, figure 4(a) shows the case in which the reversible assembly of the plate 4 to the component 3 occurs due to the insertion of at least one mushroom-shaped projection 8, to be inserted inside at least one corresponding slit 9, present on the surface of the component 3 to be directed towards the muscular floor of the mouth of the patient. Figure 4(b) instead shows a plan view of the surface 5' of the plate 4 on which at least one slit 11 is present, adapted to receive at least one corresponding projection 8 placed on the surface 5 of a further plate 4 to be assembled to the first. This embodiment allows obtaining a device 1 comprising the mandibular realignment element having a sandwich structure.

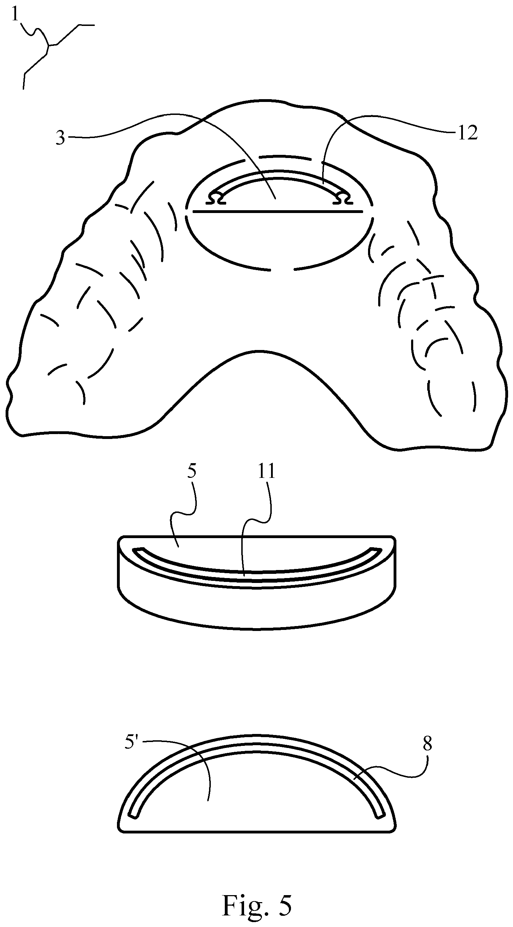

FIGURE 5 shows a view of an embodiment similar to that described in figure 4, but referred to an intraoral device 1, in which it is the component 3 which has at least one mushroom-shaped projection 12, while the surface 5 of the component 4 has a corresponding slit 11 adapted for the reversible assembly. The figure also shows the case in which the projection 12, the projection 8 and the slit 11 have an arch-like extension.

### Descriptions of the preferred embodiments

The intraoral device 1 for improving balance, posture and morphofunctional alignment of the craniomandibular complex, object of the present document, comprises: a sheath 2, made of polymeric material and preferably of resin, to be applied to at least one portion of the upper dental arch of the subject using said device 1. In a particular embodiment, said sheath 2 is extended over the entire upper dental arch of the buccal system of the subject using the device 1. Alternatively, said sheath 2 can be only be partially extended on the upper dental arch of the subject, or it can cover the entire upper dental arch, and project from the latter, being extended along the entire palate of the subject. In this case, the sheath 2 will have an extended portion 10 that will constantly contact the palate of the subject. In a particular embodiment of the present invention, said extended portion 10 has at least one hole 13 adapted to ensure that the presence of the same extended portion 10 does not interfere with palatal respiration.

The selection of the sheath 2 will of course depend on the type of dysfunction encountered in the patient. The device 1 further comprises a palate stimulation component 3 represented by a portion firmly integrated with the sheath 2, with which said component 3, with thickness comparable to that of the height of the incisors of the upper dental arch, constitutes a single body; such component is extended, starting from the back of said upper incisors, along the front portion of the palate, when said device 1 is worn by the patient. Said component 3, as was already stated in the course of the present description, is adapted to exert a mild and constant pressure on the front portion of the palate, stimulating, in a more physiological manner, the receptors involved in the transmission of postural information through the trigeminal nerve. Said component 3 further represents a surface of occlusion for the incisors of the lower dental arch. This prevents both the molars and premolars from being involved in buccal clenching, preventing possible contractures and consequent dysfunctional responses. The present intraoral device 1 further comprises at least one mandibular realignment plate 4, with variable geometry, to be assembled to the sheath 2. For example, the plate 4 can have semidiscoid geometry, with the curvature directed towards the outside of the buccal system. More in detail, the assembly involves the plate 4 and the palate stimulation component 3 incorporated with the sheath 2. The assembly of the plate 4 to the component 3 occurs with different methods, and in some cases depending on the specific applications for which a determinate therapeutic path is requested. In order to more clearly illustrate the invention, it is convenient to indicate the presence of two surfaces of the plate 4, i.e. the surface 5, to be directed towards the palate, and the surface 5', opposite the surface 5 and to be directed towards the muscular floor of the mouth of the subject. In a particular embodiment of the invention, the plate 4 has, on the surface thereof 5 to be directed towards the palate, at least one pin 6, and preferably three pins 6 firmly fixed to the plate 4, projecting therefrom, and to be inserted in corresponding holes 7 present on the surface of the component 3 which is of course directed towards the muscular floor of the mouth. In another embodiment, the assembly of the plate 4 to the component 3 occurs by means of gluing. In this case the present device 1 is provided with a common fixing paste used for intraoral medical device applications.

In still another embodiment, the assembly of the plate 4 to the component 3 occurs with a pressure fitting mechanism. For such purpose, the plate 4 has on the surface thereof 5 at least one mushroom-shaped projection, preferably but not necessarily obtained in the same material constituting said plate 4, adapted to be fit inside a corresponding slit present on the component 3. More in detail in this particular embodiment, the device 1 comprises the plate/plates 4 having at least one mushroom-shaped projection 8, adapted to be stably inserted and fixed, with a pressure fitting mechanism, inside at least one corresponding slit 9 placed on the surface of the component 3, to be directed towards the muscular floor of the mouth of the subject. The surface 5' of the plate/plates 4 instead has/have at least one slit 11 adapted to allow the fitting of projections 8 present on a further plate 4 to be assembled to the first, already assembled to said component 3. This embodiment is particularly convenient when it is desired to vary the thickness of the plate, if plates of relatively small thickness are requested. The possibility to obtain a plate having a sandwich structure is for example very convenient when the therapeutic path initially provides for the use of a plate of relatively large thickness, e.g. 1 cm, which is progressively reduced, monitoring the mandibular realignment as improvements are seen of the balance, posture and morphofunctional alignment of the craniomandibular complex of the subject.

Thus, in this embodiment each plate 4 will have on the surface thereof 5 at least one mushroom-shaped projection 8, and on the surface thereof 5' at least one slit 11 adapted for the assembly of a further plate 4. Alternatively, and in another embodiment, the arrangement of the projection 8 and the slit 11 on the plate 4 is reversed: i.e. each plate 4 will have, on the surface thereof 5, at least one slit 11, and on the surface thereof 5' at least one projection 8. Of course, in the latter case the surface of the component 3, to be directed towards the muscular floor of the mouth, will have at least one mushroom-shaped projection 12. The extension of the projection 8, or of the projection 12, is variable and can be, by way of a nonlimiting example, needle-like or arch-like. Analogously, the slits 9 or 11 will have an extension complementary to that of the projections. The assembly of the plate 4 to the component 3 is optional and advisable, particularly if it is necessary to induce a realignment of the symmetric mandibular alignment, with consequent improvement of peripheral physiological functions, which had been altered through the trigeminal nerve by asymmetries involving, for example, the temporomandibular joint.

In a further embodiment of the invention, the intraoral device 1 comprises at least one plate 4 which has, in its thickness, at least one channel 14, and preferably three channels 14, with diameter comprised between 0.1 cm and 1 cm, adapted to facilitate the respiration of the subject when the latter wears the device 1 provided with plate. This embodiment is particularly useful during sports fatigue. For example, in a particular embodiment of the described invention, the device 1 comprises at least one plate 4 having a channel 14 with diameter of 0.5 cm, and in another embodiment, at least three channels 14 having diameter of 0.2 cm. The thickness of the plate is variable; in particular it varies from 0.1 cm to 2 cm and preferably from 0.5 cm to 1.5 cm. For example in one of the preferred embodiments of the intraoral device 1 according to the present invention, said device 1 comprises at least one plate 4 with thickness equal to 1.5 cm, at least one plate 4 with thickness equal to 1 cm and at least one plate 4 with thickness equal to 0.1 cm. The length of the plate 4, intended from canine to canine, instead varies from 3 cm to 4 cm and is preferably equal to 3.5 cm. Said plate 4, when assembled to the palate stimulation component 3, is in fact extended on the upper dental arch of the subject, from canine to canine, without involving the molars and premolars of said subject using the device 1. The material constituting the plate 4 can also vary, along with that constituting the entire intraoral device 1. More in detail, the material constituting the intraoral device 1 must first of all have characteristics that are absolutely harmless for the human body, i.e. it must be non-toxic, non-allergenic, not too hard, not too soft, not imbibing and preferably flavorless. Materials that are well adapted for the application in question are copolymer materials with high molecular weight comprising, as monomer units, ethylene and vinyl acetate. In particular, by varying the vinyl acetate percentage, it is possible to obtain a product with specific application requirements, and with optical characteristics like semi-transparency and opacity. Another material suitable for making the intraoral device 1, and which is widely used today for the manufacturing of dental prostheses, is a polymer commonly known as plastulene. This is a thermoplastic polymer, in particular a modified polyethylene that allows obtaining structures having flexible edges, giving the wearer of the prosthesis an increased seal both in rest phase and work phase of the prosthesis. A recent development of plastulene provides for the addition, within the polymer, of titanium particles. Thermo-adaptable polymers can also be used for obtaining the present intraoral device 1, such polymers recently employed in dentistry. Such materials have the capacity to be deformed when subjected to temperatures such as those that can be found inside the buccal system. The advantage offered by this characteristic is that of making the components of the device in question more adaptable to the buccal system of the subject, rendering said device (1) even more suitable. At any rate, for the obtainment of the intraoral device 1 according to the present invention, any polymeric material is adapted which is non-mutagenic, non-cytotoxic and biocompatible.

## Claims

1. Intraoral device (1) for improving balance, posture and morphofunctional alignment of the craniomandibular complex comprising a sheath (2) adapted to cover at least one portion of the upper dental arch of the subject using said device (1), **characterized in that** it comprises the palate stimulation component (3) adapted to induce a constant stimulation of the receptors present in the front portion of the palate simulating lingual activity, said component (3) being firmly integrated with said sheath (2) and being extended, from the back of the upper incisors, along the front portion of the palate when said device (1) is worn, and **in that** it comprises at least one plate (4) with variable thickness and geometry, suitable for mandibular realignment, and to be assembled to said sheath (2), said plate (4) having the surface (5) to be directed towards the palate, and the surface (5') to be directed towards the muscular floor of the mouth of the subject, and being reversibly assemblable to the palate stimulation component (3) integral with said sheath (2).

2. Intraoral device (1) for improving balance, posture and morphofunctional alignment of the craniomandibular complex according to the preceding claim, **characterized in that** the sheath (2) is partially extended on the upper dental arch of the subject, or over the entire upper dental arch, or over the entire upper dental arch projecting therefrom and being extended along the palate of the subject, said sheath (2) having, in the latter case, the extended portion (10) adapted to constantly contact the palate of the subject when said device (1) is worn.

3. Intraoral device (1) for improving balance, posture and morphofunctional alignment of the craniomandibular complex according to the preceding claims, **characterized in that** the mandibular realignment plate (4) is extended from canine to canine when said plate (4) is assembled to said component (3) and when said device (1) is applied to the buccal system of the user subject.

4. Intraoral device (1) for improving balance, posture and morphofunctional alignment of the craniomandibular complex, according to the preceding claims, **characterized in that** the reversible assembly of said plate (4) to said component (3) occurs due to the presence of at least one pin (6), integral with the plate (4), to be inserted inside at least one corresponding hole (7) present on the component (3), said component (4) having said pin/pins (6) projecting from the surface (5) of the plate (4), to be directed towards the palate of the subject when said device (1) is worn, and said component (3) having the hole/holes (7) on the surface thereof directed towards the muscular floor of the buccal system of the subject using the device (1).

5. Intraoral device (1) for improving balance, posture and morphofunctional alignment of the craniomandibular complex according to claims 1 to 3, **characterized in that** the reversible assembly of said plate (4) to said component (3) occurs by means of gluing, said device (1) being provided with a common fixing paste for intraoral medical device applications.

6. Intraoral device (1) for improving balance, posture and morphofunctional alignment of the craniomandibular complex according to claims 1 to 3, **characterized in that** the reversible assembly of the plate (4) to the component (3) occurs due to the presence of at least one mushroom-shaped projection (8), present on the surface (5) of said plate (4), to be inserted inside at least one corresponding slit (9) placed on the surface of the component (3), to be directed towards the muscular floor of the mouth and vice versa, said component/components (4) alternatively having at least one slit (11) on the surface (5) to be assembled with at least one corresponding projection (12) present on the surface of the component (3) to be directed towards the muscular floor of the mouth, and **in that** the plate/plates (4) has/have, on the surface thereof (5'), at least one slit (11) or one projection (8) adapted for the assembly of at least one corresponding projection (8) or slit (11) present on the surface (5) of a further plate (4) to be assembled to the first, said device (1) having the mandibular realignment component with sandwich structure.

7. Intraoral device (1) for improving balance, posture and morphofunctional alignment of the craniomandibular complex according to any one of the preceding claims, **characterized in that** the mandibular realignment plate/plates (4) has/have a thickness comprised between 0.1 cm and 2 cm.

8. Intraoral device (1) for improving balance, posture and morphofunctional alignment of the craniomandibular complex according to claim 2, **characterized in that** the extended portion (10), when present, has at least one hole (13) adapted to facilitate the respiration of the palate.

9. Device (1) for improving balance, posture and morphofunctional alignment of the craniomandibular complex according to any one of the preceding claims, **characterized in that** the plate/plates (4) has/have at least one channel (14), with diameter comprised between 0.1 cm and 1 cm, adapted to facilitate the respiration of the subject.

10. Intraoral device (1) for improving balance, posture and morphofunctional alignment of the craniomandibular complex according to any one of the preceding claims, **characterized in that** the plate/plates (4) has/have a length comprised between 3 cm and 4 cm at the distance between the two upper canines of the buccal system of the subject using the device (1).

11. Intraoral device (1) for improving balance, posture and morphofunctional alignment of the craniomandibular complex according to any one of the preceding claims, **characterized in that** it is made of a polymeric material that is non-toxic for the human body and biocompatible.

12. Intraoral device (1) for improving balance, posture and morphofunctional alignment of the craniomandibular complex according to any one of the preceding claims, **characterized in that** it is made of a polymeric material based on ethylene and vinyl acetate or polyethylene added with titanium and/or a material thermo-adaptable to the buccal system of the subject.

## Patentansprüche

1. Intraorale Vorrichtung (1) zur Verbesserung des Gleichgewichts, der Haltung und der morphofunktionellen Ausrichtung des kraniomandibulären Komplexes, eine Auflage (2) umfassend, die dafür ausgelegt ist, mindestens einen Abschnitt des oberen Zahnbogens eines Behandelten, der die Vorrichtung (1) nutzt, zu bedecken, **dadurch gekennzeichnet, dass** sie eine Gaumenstimulierungskomponente (3) umfasst, die dafür ausgelegt ist, eine ständige Stimulierung der Rezeptoren hervorzurufen, die im vorderen Abschnitt des Gaumens vorhanden sind, um eine linguale Aktivität zu simulieren, wobei die Komponente (3) ein integraler Bestandteil der Auflage (2) ist und sich, wenn die Vorrichtung (1) eingesetzt ist, von der Rückseite der oberen Schneidezähne aus entlang des vorderen Abschnitts des Gaumens erstreckt, und dadurch, dass sie mindestens eine Platte (4) mit variabler Dicke und Geometrie umfasst, die sich für eine Neuausrichtung des Unterkiefers eignet und die an der Auflage (2) zu montieren ist, wobei die Platte (4) eine auf den Gaumen zu richtende Oberfläche (5) und eine auf die Muskulatur des Mundbodens des Behandelten zu richtende Oberfläche (5') aufweist und reversibel an der in die Auflage (2) integrierten Gaumenstimulierungskomponente (3) montierbar ist.

2. Intraorale Vorrichtung (1) zur Verbesserung des Gleichgewichts, der Haltung und der morphofunktionellen Ausrichtung des kraniomandibulären Komplexes nach dem vorangehenden Anspruch, **dadurch gekennzeichnet, dass** sich die Auflage (2) teilweise am oberen Zahnbogen des Behandelten oder über dem gesamten oberen Zahnbogen entlang erstreckt oder sich über dem gesamten oberen Zahnbogen erstreckt und über diesen vorsteht und entlang des Gaumens des Behandelten erweitert ist, wobei im letztgenannten Fall ein erweiterter Abschnitt (10) der Auflage (2) dafür ausgelegt ist, ständig Kontakt mit dem Gaumen des Behandelten zu halten, wenn die Vorrichtung (1) eingesetzt ist.

3. Intraorale Vorrichtung (1) zur Verbesserung des Gleichgewichts, der Haltung und der morphofunktionellen Ausrichtung des kraniomandibulären Komplexes nach den vorangehenden Ansprüchen, **dadurch gekennzeichnet, dass** sich die der Neuausrichtung des Unterkiefers dienende Platte (4) von Eckzahn zu Eckzahn erstreckt, wenn die Platte (4) an der Komponente (3) montiert ist und wenn die Vorrichtung (1) am bukkalen System des Behandelten, der sie nutzt, angebracht ist.

4. Intraorale Vorrichtung (1) zur Verbesserung des Gleichgewichts, der Haltung und der morphofunktionellen Ausrichtung des kraniomandibulären Komplexes nach den vorangehenden Ansprüchen, **dadurch gekennzeichnet, dass** die reversible Montage der Platte (4) an der Komponente (3) durch das Vorhandensein mindestens eines Stiftes (6) vonstatten geht, der eine Einheit mit der Platte (4) bildet und der in mindestens ein entsprechendes Loch (7) einzuführen ist, das an der Komponente (3) vorhanden ist, wobei der Stift / die Stifte (6) an der Komponente (4) von der Oberfläche (5) der Platte (4) vorsteht / vorstehen, so dass er / sie auf den Gaumen des Behandelten gerichtet ist / sind, wenn die Vorrichtung (1) getragen wird, und das Loch /die Löcher (7) an der Oberfläche der Komponente (3) auf die Muskulatur des Mundbodens des Behandelten, der die Vorrichtung (1) nutzt, gerichtet ist / sind.

5. Intraorale Vorrichtung (1) zur Verbesserung des Gleichgewichts, der Haltung und der morphofunktionellen Ausrichtung des kraniomandibulären Komplexes nach den Ansprüchen 1 bis 3, **dadurch gekennzeichnet, dass** die reversible Montage der Platte (4) an der Komponente (3) durch Kleben vonstattengeht, wobei die Vorrichtung (1) mit einer üblichen Fixierpaste für die Anbringung intraoraler medizinischer Vorrichtungen versehen wird.

6. Intraorale Vorrichtung (1) zur Verbesserung des Gleichgewichts, der Haltung und der morphofunktionellen Ausrichtung des kraniomandibulären Komplexes nach den Ansprüchen 1 bis 3, **dadurch gekennzeichnet, dass** die reversible Montage der Platte (4) an der Komponente (3) aufgrund des Vorhandenseins mindestens eines pilzförmigen Vorsprungs (8) vonstattengeht, der an der Oberfläche (5) der Platte (4) vorhanden ist und der in mindestens einen entsprechenden, an der Oberfläche der Komponente (3) angeordneten Schlitz (9) einzuführen ist, der auf die Muskulatur des Mundbodens zu richten ist, und umgekehrt, wobei die Komponente / die Komponenten (4) alternativ an der Oberfläche (5) dazu mindestens einen Schlitz (11) aufweist/aufweisen, der mit mindestens einem entsprechenden, an der Oberfläche der Komponente (3) vorhandenen und auf die Muskulatur des Mundbodens zu richtenden Vorsprung (12) zusammenzusetzen ist, und dadurch, dass die Platte / die Platten (4) auf der Oberfläche (5') mindestens einen Schlitz (110) oder einen Vorsprung (8) aufweisen, der zum Zusammensetzen mit mindestens einem entsprechenden Vorsprung (8) oder Schlitz (11), der an der Oberfläche (5) einer weiteren, mit der ersteren zusammenzusetzenden Platte (4) vorhanden ist, ausgelegt ist, wobei die Vorrichtung (1) die Kieferneuausrichtungskomponente mit Sandwich-Struktur aufweist.

7. Intraorale Vorrichtung (1) zur Verbesserung des Gleichgewichts, der Haltung und der morphofunktionellen Ausrichtung des kraniomandibulären Komplexes nach einem der vorangehenden Ansprüche, **dadurch gekennzeichnet, dass** die Kieferneuausrichtungsplatte /-platten (4) eine Dicke zwischen 0,1 cm und 2 cm aufweist / aufweisen.

8. Intraorale Vorrichtung (1) zur Verbesserung des Gleichgewichts, der Haltung und der morphofunktionellen Ausrichtung des kraniomandibulären Komplexes nach Anspruch 2, **dadurch gekennzeichnet, dass** der erweiterte Abschnitt (10), falls vorhanden, mindestens ein Loch (13) aufweist, das dafür ausgelegt ist, die Belüftung des Gaumens zu verbessern.

9. Vorrichtung (1) zur Verbesserung des Gleichgewichts, der Haltung und der morphofunktionellen Ausrichtung des kraniomandibulären Komplexes nach einem der vorangehenden Ansprüche, **dadurch gekennzeichnet, dass** die Platte / die Platten (4) mindestens einen Kanal (14) mit einem Durchmesser zwischen 0,1 cm und 1 cm aufweist / aufweisen, der dafür ausgelegt ist, die Respiration des Behandelten zu erleichtern.

10. Intraorale Vorrichtung (1) zur Verbesserung des Gleichgewichts, der Haltung und der morphofunktionellen Ausrichtung des kraniomandibulären Komplexes nach einem der vorangehenden Ansprüche, **dadurch gekennzeichnet, dass** die Platte / die Platten (4) am Abstand zwischen den beiden oberen Eckzähnen des bukkalen Systems des Behandelten, der die Vorrichtung (1) nutzt, eine Länge zwischen 3 cm und 4 cm aufweist / aufweisen.

11. Intraorale Vorrichtung (1) zur Verbesserung des Gleichgewichts, der Haltung und der morphofunktionellen Ausrichtung des kraniomandibulären Komplexes nach einem der vorangehenden Ansprüche, **dadurch gekennzeichnet, dass** sie aus einem polymeren Material besteht, das ungiftig für den menschlichen Körper und biokompatibel ist.

12. Intraorale Vorrichtung (1) zur Verbesserung des Gleichgewichts, der Haltung und der morphofunktionellen Ausrichtung des kraniomandibulären Komplexes nach einem der vorangehenden Ansprüche, **dadurch gekennzeichnet, dass** sie aus einem polymeren Material auf Basis von Ethylen und Vinylacetat oder Polyethylen besteht, dem Titan und/oder ein Material zugesetzt wurde, das sich thermisch an das bukkale System des Behandelten anpassen kann.

## Revendications

1. Dispositif intra-buccal (1) pour améliorer l'équilibre, la position et l'alignement morpho-fonctionnel du complexe cranio-mandibulaire comprenant une gaine (2) adaptée pour couvrir au moins une partie de l'arc dentaire supérieur du sujet en utilisant ledit dispositif (1), **caractérisé en ce qu'**il comprend le composant de stimulation palatal (3) adapté pour induire une stimulation constante des récepteurs présents dans la partie avant du palais stimulant l'activité linguale, ledit composant (3) étant solidement intégré dans ladite gaine (2) et étant étendu, à partir de l'arrière des incisives supérieures, le long de la partie avant du palais lorsque ledit dispositif (1) est porté, et **en ce qu'**il comprend au moins une plaque (4) présentant une épaisseur et une géométrie variables, appropriée pour un réalignement mandibulaire, et pour être assemblée à ladite gaine (2), ladite plaque (4) comportant la surface (5) à diriger vers le palais, et la surface (5') à diriger vers le plancher musculaire de la bouche du sujet, et pouvant étant assemblée de façon réversible au composant de stimulation palatal (3) d'un seul tenant avec ladite gaine (2).

2. Dispositif intra-buccal (1), pour améliorer l'équilibre, la position et l'alignement morpho-fonctionnel du complexe cranio-mandibulaire selon la revendication précédente, **caractérisé en ce que** la gaine (2) est partiellement étendue sur l'arc dentaire supérieur du sujet, ou sur l'arc dentaire supérieur entier, ou sur l'arc dentaire supérieur entier se projetant depuis celle-ci et étant étendu le long du palais du sujet, ladite gaine (2) comportant dans ce dernier cas, la partie étendue (10) adaptée pour être constamment en contact avec le palais du sujet lorsque ledit dispositif (1) est porté.

3. Dispositif intra-buccal (1), pour améliorer l'équilibre, la position et l'alignement morpho-fonctionnel du complexe cranio-mandibulaire selon la revendication précédente, **caractérisé en ce que** la plaque de réalignement mandibulaire (4) est étendue d'une canine à l'autre lorsque ladite plaque (4) est assemblée audit composant (3) et lorsque ledit dispositif (1) est appliqué au système bucco-dentaire de l'utilisateur en question.

4. Dispositif intra-buccal (1), pour améliorer l'équilibre, la position et l'alignement morpho-fonctionnel du complexe cranio-mandibulaire selon les revendications précédentes, **caractérisé en ce que** l'assemblage réversible de ladite plaque (4) audit composant (3) s'effectue en raison de la présence d'au moins une broche (6), d'un seul tenant avec la plaque (4), à insérer à l'intérieur d'au moins un trou correspondant (7) présent sur le composant (3), ledit composant (4) comportant ladite broche/ lesdites broches (6) se projetant de la surface (5) de la plaque (4) pour être dirigée vers le palais du sujet lorsque ledit dispositif (1) est porté, et ledit composant (3) comportant le/ les trou(s) (7) sur la surface de celui-ci dirigé vers le plancher musculaire du système bucco-dentaire du sujet utilisant le dispositif (1).

5. Dispositif intra-buccal (1), pour améliorer l'équilibre, la position et l'alignement morpho-fonctionnel du complexe cranio-mandibulaire selon les revendications 1 à 3, **caractérisé en ce que** l'ensemble réversible de ladite plaque (4) audit composant (3) s'effectue au moyen d'un collage, ledit dispositif (1) étant doté d'une pâte de fixation courante pour des applications de dispositifs médicaux intra-buccaux.

6. Dispositif intra-buccal (1), pour améliorer l'équilibre, la position et l'alignement morpho-fonctionnel du complexe cranio-mandibulaire selon les revendications 1 à 3, **caractérisé en ce que** l'ensemble réversible de la plaque (4) au composant (3) s'effectue grâce à la présence d'au moins une projection (8) en forme de champignon présente sur la surface (5) de ladite plaque (4), à insérer à l'intérieur d'au moins une fente correspondante (9) placée sur la surface du composant (3), pour être dirigée vers le plancher musculaire de la bouche et inversement, ledit composant/ lesdits composants (4) comportant en variante au moins une fente (11) sur la surface (5) à assembler avec au moins une projection correspondante (12) présente sur la surface du composant (3) à diriger vers le plancher musculaire de la bouche, et **en ce que** la/les plaque(s) (4) comporte(nt), sur leur surface (5'), au moins une fente (11) ou une projection (8) adaptée pour l'assemblage d'au moins une projection (8) ou fente (11) correspondante présente sur la surface (5) d'une autre plaque (4) à assembler à ladite première, ledit dispositif (1) comportant le composant de réalignement mandibulaire présentant une structure en sandwich.

7. Dispositif intra-buccal (1), pour améliorer l'équilibre, la position et l'alignement morpho-fonctionnel du complexe cranio-mandibulaire selon l'une quelconque des revendications précédentes, **caractérisé en ce que** la/les plaque(s) de réalignement mandibulaire (4) présente(nt) une épaisseur comprise entre 0,1 cm et 2 cm.

8. Dispositif intra-buccal (1), pour améliorer l'équilibre, la position et l'alignement morpho-fonctionnel du complexe cranio-mandibulaire selon la revendication 2, **caractérisé en ce que** la partie étendue (10), lorsqu'elle est présente, au moins un trou (13) adapté pour faciliter la respiration du palais.

9. Dispositif (1), pour améliorer l'équilibre, la position et l'alignement morpho-fonctionnel du complexe cranio-mandibulaire selon l'une quelconque des revendications précédentes, **caractérisé en ce que** la/les plaque(s) (4) comporte(nt) au moins un canal (14), présentant un diamètre compris entre 0,1 cm et 1 cm, adapté pour faciliter la respiration du sujet.

10. Dispositif intra-buccal (1), pour améliorer l'équilibre, la position et l'alignement morpho-fonctionnel du complexe cranio-mandibulaire selon l'une quelconque des revendications précédentes, **caractérisé en ce que** la/les plaque(s) (4) présente(nt) une longueur comprise entre 3 cm et 4 cm à la distance entre les deux canines supérieures du système bucco-dentaire du sujet utilisant le dispositif (1).

11. Dispositif intra-buccal (1), pour améliorer l'équilibre, la position et l'alignement morpho-fonctionnel du complexe cranio-mandibulaire selon l'une quelconque des revendications précédentes, **caractérisé en ce qu'**il est constitué d'un matériau polymère qui est non toxique pour le corps humain et biocompatible.

12. Dispositif intra-buccal (1), pour améliorer l'équilibre, la position et l'alignement morpho-fonctionnel du complexe cranio-mandibulaire selon l'une quelconque des revendications précédentes, **caractérisé en ce qu'**il est constitué d'un matériau polymère à base d'éthylène et d'acétate de vinyle ou de polyéthylène additionné de titane et/ou d'un matériau thermo-adaptable au système bucco-dentaire du sujet.
